# EUROPEAN PATENT SPECIFICATION

(11) **EP 4 241 077 B1**
(45) Date of publication and mention of the grant of the patent: **21.01.2026**
(21) Application number: 20960968.4
(22) Date of filing: 06.11.2020
(51) Int. Cl.: G01N 33/18, G01N 21/64, C02F 1/24, C02F 1/28, C02F 1/44, C02F 9/00

(54) **METHOD AND SYSTEM FOR DETERMINING SURFACTANT CONCENTRATION IN INDUSTRIAL PROCESSES**
VERFAHREN UND SYSTEM ZUR BESTIMMUNG DER TENSIDKONZENTRATION IN INDUSTRIELLEN PROZESSEN
PROCÉDÉ ET SYSTÈME DE DÉTERMINATION DE LA CONCENTRATION EN AGENTS TENSIOACTIFS DANS DES PROCESSUS INDUSTRIELS

(43) Date of publication of application: 13.09.2023
(73) Proprietor: Kimberly-Clark Worldwide, Inc., Neenah, Wisconsin 54956 (US)
(72) Inventor: POLASHOCK, Vicky, Neehan, Wisconsin 54956 (US); DHAGUMUDI, Vetrivel, Neehan, Wisconsin 54956 (US); FRASER, Brian S., Neehan, Wisconsin 54956 (US)
(74) Representative: Dehns
(86) International application number: PCT/US2020/059352
(87) International publication number: WO 2022/098362

(56) References cited:
- EP-A1- 2 799 866
- JP-A- 2017 227 577
- US-A1- 2011 036 271
- US-A1- 2015 260 702
- US-B1- 6 287 417
- US-B2- 9 476 866

## Description

### BACKGROUND

Surfactants are used in all different types of industrial processes. Almost all surfactants are organic in nature and are amphiphilic including hydrophobic groups and hydrophilic groups. Types of surfactants include nonionic surfactants, anionic surfactants, cationic surfactants, and zwitterionic surfactants. Surfactants are also known as surface-active agents that can reduce surface tension. When added to a liquid, a surfactant can improve the wetting properties of other components that contact the liquid. Surfactants can serve as emulsifying agents, wetting agents, foaming agents, and the like.

For example, during the production of paper products and tissue products, surfactants can be added to fluids at different points in the process. In order to produce paper products and tissue products, for instance, pulp fibers are typically combined with water and applied to a forming surface for forming a web. Surfactants can be added to the aqueous suspension of fibers in order to improve the wetting characteristics of the fibers and/or to better disperse the fibers uniformly within the aqueous suspension. In one particular type of process, the pulp fibers are suspended in a foam that is then used to form the web. In this application, the surfactant can serve as the foaming agent in order to produce the foam-formed web.

During various different industrial processes that utilize surfactants, it is desirable to know with some precision the surfactant concentration within the aqueous solution. For example, during certain industrial processes, surfactant concentration should be within a desired range in order to optimize the process. For example, a certain minimum level of surfactant may be needed in some processes in order to make high-quality products. At other points in the process, controls may need to be put in place in order to ensure that surfactant levels are below a desired level so that, for instance, wastewater produced during the process can be discharged to the environment. Maintaining surfactant levels below certain concentrations, for instance, may be necessary in order to ensure compliance with environmental regulatory standards.

In the past, the measurement of surfactant levels was conducted off-line. Chemical analysis also took several hours or days to complete. The long delay in being able to test for surfactant levels has limited the ability of industrial processes to make rapid adjustments in process conditions on the fly. In addition, surfactant chemicals can be relatively expensive. Based on inefficient testing regimes, surfactant over-dosing and waste has also been a problem. US 2011/036271 A1 discloses a method for determining surfactant levels in a fluid.

In view of the above, a need currently exists for a method and system that can accurately and rapidly determine surfactant concentration levels in aqueous solutions, particularly in industrial water streams.

### SUMMARY

In general, the present disclosure is directed to a method and system for determining surfactant concentrations in an aqueous solution. The aqueous solution can be part of a fluid stream in an industrial process, such as a process for producing paper products and/or absorbent articles. The method and system of the present disclosure, however, has broad applicability in all fields and in all different types of process designs. Surfactant concentrations can be determined rapidly for then making changes or adjustments within the process for selectively increasing or decreasing surfactant concentrations in order to maintain surfactant concentrations below a preset limit or within preset limits.

For example, in one embodiment, the present disclosure is directed to a method for determining surfactant levels in a fluid. The method includes collecting a fluid sample from an aqueous solution. The aqueous solution contains a surfactant. The aqueous solution, for instance, can be a fluid stream in an industrial process, such as a papermaking, a tissue making process, a process for making other nonwoven materials or substrates, or a process associated with making absorbent articles, such as personal care products including diapers, feminine care products, adult incontinence products or the like. A carbon related parameter is then measured from the fluid sample. The carbon related parameter, for example, can be Chemical Oxygen Demand (COD) or Total Organic Carbon (TOC). In accordance with the present disclosure, the surfactant concentration within the fluid sample is then determined by comparing the measured carbon related parameter to a calibration data set that indicates surfactant concentration based on the measured carbon related parameter. The calibration data set, for instance, can be a calibration curve that may have been constructed based on reference data.

Based on the determined surfactant concentration, the method of the present disclosure may further include the step of increasing or decreasing surfactant concentration in the aqueous solution in order to maintain the surfactant concentration within a preset limit. For example, the surfactant concentration can be decreased for maintaining the surfactant concentration below a preset limit. In an alternative embodiment, the surfactant concentration can be selectively increased or decreased so that the surfactant concentration stays above a first preset limit and below a second preset limit. In one embodiment, a controller can be used to receive the measurement of the carbon related parameter and can then calculate or determine the surfactant concentration. The controller can also be configured to automatically increase or decrease surfactant concentrations within the aqueous solution based upon the determined surfactant concentration value. For example, the controller can be configured to increase or decrease the flow rate of a surfactant into the aqueous solution from a surfactant supply or can be configured to increase or decrease the flow of water into the aqueous solution from a water supply.

In accordance with the present disclosure, the surfactant concentration can be determined rapidly, efficiently, and in-line (e.g. automatic sampling and measurement). For example, in one aspect, the surfactant concentration of the aqueous solution can be measured about every 12 hours, such as about every 6 hours, such as about every 4 hours, such as about every 2 hours, such as about every hour. Rapid determination of surfactant concentration allows for quick and instantaneous adjustments to the process to maintain surfactant levels within the preset limits.

In general, any suitable surfactant can be monitored in accordance with the present disclosure. The surfactant, for instance, can be a nonionic surfactant, an anionic surfactant, a cationic surfactant, or a zwitterionic surfactant. Examples of particular surfactants that may be monitored in accordance with the present disclosure include sodium dodecyl sulfate, ammonium lauryl sulfate, a fatty acid amine, an amine oxide, a fatty acid quaternary compound, or lauryl sulfate. In one aspect, the surfactant contained in the aqueous solution is an alkyl polyglycoside.

The method and system of the present disclosure are particularly well suited for monitoring surfactant concentrations in a papermaking or tissue making process or a process associated with making absorbent articles, such as personal care products. For example, in one embodiment, the aqueous solution can be fed to a headbox for forming a suspension of fibers, such as a foamed suspension of fibers. The aqueous solution can be formed by combining water from a water supply with a surfactant from a surfactant supply. In accordance with the present disclosure, based upon the determined surfactant concentration, a controller can be used to selectively increase or decrease the amount of surfactant being fed from the surfactant supply into the aqueous solution for maintaining surfactant concentration within preset limits. Alternatively, the flow of water from the water supply can be selectively increased or decreased for maintaining surfactant concentrations within preset limits.

In embodiments according to the claimed invention, the method and system of the present disclosure can be used to ensure that surfactant concentrations are maintained below certain limits in order to discharge the aqueous solution to the environment or otherwise recycle the aqueous stream. In these embodiments, the aqueous solution is first fed through a separating device that removes surfactant from the aqueous solution prior to collecting the fluid sample. The separating device, for instance, can use dissolved ozone flotation, can be any suitable filter such as an activated carbon filter, and/or can use reverse osmosis in order to remove surfactant from the aqueous solution. The separating device can produce a surfactant poor stream comprising the aqueous solution that is sampled and a surfactant rich stream. The surfactant concentrations can be monitored in the surfactant poor stream and, if surfactant levels are maintained below certain levels, be fed to an effluent. If surfactant levels in the surfactant poor stream are above a preset limit, on the other hand, the surfactant poor stream can be fed back to the separating device for decreasing surfactant concentration within the aqueous solution.

Prior to feeding the aqueous solution to the separating device, the aqueous solution can also be filtered for suspended solids. For instance, solids can be removed from the aqueous solution using dissolved air flotation, suspended air flotation, or any suitable filter.

The present disclosure is also directed to a system for measuring surfactant concentration in a water supply. The system includes a carbon parameter analyzer that measures Chemical Oxygen Demand or Total Organic Carbon in a fluid sample. The carbon parameter analyzer is configured to receive a fluid sample from an industrial fluid stream. The industrial fluid stream comprises an aqueous solution containing a surfactant. The system further includes a controller in communication with the carbon parameter analyzer. The controller is configured to receive the measured Chemical Oxygen Demand or Total Organic Carbon parameter and, determine a surfactant concentration from the measurement. The surfactant concentration, for instance, can be determined from reference data that may be stored in the controller. The controller can be further configured to selectively increase or decrease surfactant concentrations within the aqueous solution by controlling the flow of at least one fluid stream within the industrial process.

In one aspect, the carbon parameter analyzer can measure the carbon related parameter using ferrate oxidation. In another aspect, the carbon parameter analyzer can include a fluorescence sensor or UV sensor.

Other features and aspects of the present disclosure are discussed in greater detail below.

### BRIEF DESCRIPTION OF THE DRAWINGS

A full and enabling disclosure of the present disclosure is set forth more particularly in the remainder of the specification, including reference to the accompanying figures, in which:
Figure 1 is one embodiment of a paper making system that may include the system of the present disclosure;
Figure 2 is one embodiment of a surfactant measuring system made in accordance with the present disclosure;
Figure 3 is one embodiment of a calibration curve that may be used in accordance with the present disclosure;
Figure 4 is another embodiment of a calibration curve that may be used in accordance with the present disclosure; and

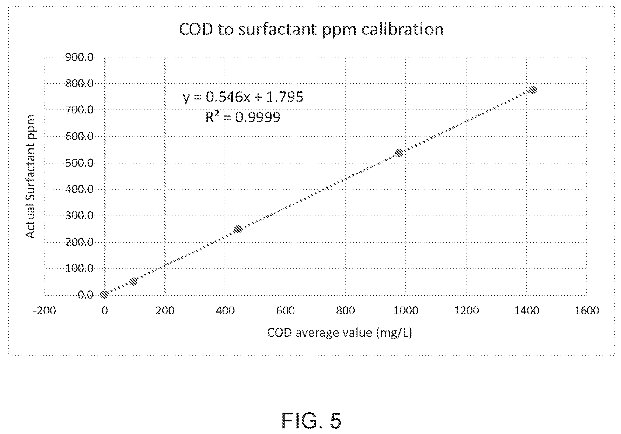

Figure 5 is still another embodiment of a calibration curve that may be used in accordance with the present disclosure.

Repeat use of reference characters in the present specification and drawings is intended to represent the same or analogous features or elements of the present invention.

### DETAILED DESCRIPTION

It is to be understood by one of ordinary skill in the art that the present discussion is a description of exemplary embodiments only and is not intended as limiting the broader aspects of the present disclosure.

Surfactants are used in all different types of production processes. Typically, the surfactants are combined with a water supply or other aqueous solution in order to provide a benefit in producing a product or in facilitating the production of a product. Being able to determine surfactant levels in production processes can provide various benefits and advantages. For example, in certain processes, surfactant levels should be maintained above a minimum level in order to optimize the process or otherwise produce a high-quality product. In other situations, however, surfactant levels need to be maintained below certain levels in order to ensure compliance with regulatory standards, such as pollutant discharge standards. Further, while surfactants are beneficial in certain processes, surfactants can cause problems in other parts of the same production facility.

The present disclosure is generally directed to a method and system for determining surfactant levels or concentration within aqueous streams rapidly and efficiently. For example, in one aspect, the system of the present disclosure can determine surfactant levels in a process fluid in real time. In addition to quickly determining surfactant concentrations in an aqueous solution, the method and system of the present disclosure is also directed to, based on the surfactant concentration determination, making adjustments and changes in the process in order to increase or decrease surfactant levels so that the surfactant concentrations stay within preset limits. Surfactant concentration, for instance, can be controlled in an open loop system or in a closed loop system.

Surfactant chemicals are also very expensive components in industrial processes. Being able to determine surfactant concentrations also eliminates waste and overuse of the chemical.

According to the present disclosure, surfactant levels in aqueous solutions, particularly process fluids, are determined indirectly. Instead of measuring surfactant levels directly, the method and system of the present disclosure measure a carbon related parameter within a fluid sample that can be an analog for surfactant concentration. The carbon related parameter, for instance, can be Chemical Oxygen Demand (COD) or Total Organic Carbon (TOC). It was discovered that surfactant levels in fluids can be correlated to COD and/or TOC measurements. COD and/or TOC measurements can be done very quickly and can even be done in-line. For instance, fluid samples can be automatically collected and tested through fluid communication with the process fluid line or tank.

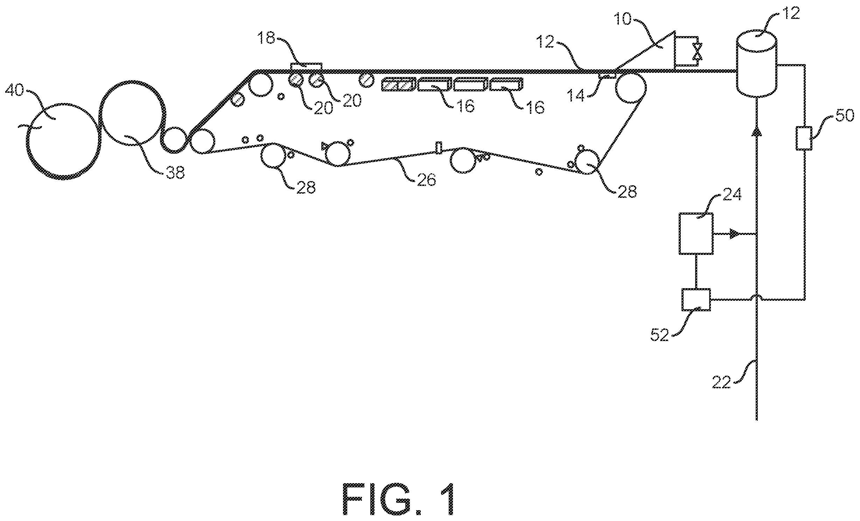

Although the method and system of the present disclosure can be used in any suitable process or environment, in one aspect, the method and system of the present disclosure can be used to control surfactant levels in any suitable process that includes an aqueous solution containing a suspension of solids, such as particulates including super absorbent materials. For example, the process can be a papermaking or tissue making process or a process for producing an absorbent article. For exemplary and demonstrative purposes only, referring to **FIG. 1****,** one embodiment of a papermaking or tissue making process is illustrated. More particularly, the process illustrated in **FIG. 1** is a partial view of a process for producing high bulk tissue products, such as facial tissue, bath tissue, paper towels, and the like.

In forming tissue webs, a fiber furnish that contains cellulosic fibers is combined with water to form an aqueous suspension of fibers. The aqueous suspension of fibers is then deposited onto a forming surface for forming a web that is then conveyed downstream, optionally subjected to further processes, and ultimately dried and wound into a roll. One or more surfactants are commonly added to the aqueous suspension of fibers in order to provide various benefits and advantages. The surfactant, for instance, can increase the wettability of the fibers and/or create a better and more uniform dispersion of fibers.

In one embodiment, the surfactant can serve as a foaming agent that forms a foam. The fibers are contained in the foam and then deposited onto the forming surface. Thus, as opposed to liquid water, the carrier is a foam for the fibers. The foam can contain a large quantity of air. In foam forming processes, less water is used to form the web and thus less energy is required in order to dry the web.

During a foam-forming process, a surfactant or foaming agent is combined with water generally in an amount greater than about 0.001% by weight, such as greater than about 0.05% by weight, such as greater than about 2% by weight, such as in an amount greater than about 5% by weight, such as in an amount greater than about 10% by weight, such as in an amount greater than about 15% by weight. One or more foaming agents are generally present in an amount less than about 50% by weight, such as in an amount less than about 40% by weight, such as in an amount less than about 30% by weight, such as in an amount less than about 20% by weight. In one aspect, surfactant concentrations can be kept at low amounts such as from 0.001% to about 1% by weight. For example, in one embodiment the surfactant levels are maintained between about 200 ppm and 2000 ppm, such as between about 300 ppm and 1000 ppm.

Once the foaming agent and water are combined, the mixture is blended or otherwise subjected to forces capable of forming a foam. A foam generally refers to a porous matrix, which is an aggregate of hollow cells or bubbles which may be interconnected to form channels or capillaries.

The foam density can vary depending upon the particular application and various factors including the fiber furnish used. In one embodiment, for instance, the foam density of the foam can be greater than about 200 g/L, such as greater than about 250 g/L, such as greater than about 300 g/L. The foam density is generally less than about 600 g/L, such as less than about 500 g/L, such as less than about 400 g/L, such as less than about 350 g/L. In one embodiment, for instance, a lower density foam is used having a foam density of generally less than about 350 g/L, such as less than about 340 g/L, such as less than about 330 g/L. The foam will generally have an air content of greater than about 30%, such as greater than about 40%, such as greater than about 50%, such as greater than about 60%. The air content is generally less than about 80% by volume, such as less than about 70% by volume, such as less than about 65% by volume.

Whether a foam-forming process or a wet-lay process, surfactants that may be incorporated into the process include various organic compounds. In one embodiment, a nonionic surfactant is used. The nonionic surfactant, for instance, may comprise an alkyl polyglycoside. In one aspect, for instance, the surfactant can be a C8 alkyl polyglycoside, a C10 alkyl polyglycoside, or a mixture of C8 and C10 alkyl polyglycosides. Other surfactants may comprise sodium dodecyl sulfate, such as sodium lauryl sulfate, sodium laureth sulfate, sodium lauryl ether sulfate, or ammonium lauryl sulfate. In other embodiments, the surfactant may comprise any suitable cationic and/or amphoteric surfactant. For instance, other surfactants include fatty acid amines, amides, amine oxides, fatty acid quaternary compounds, and the like.

Whether a foam-forming process or a wet-lay process, the aqueous solution containing the surfactant is combined with cellulosic fibers to form the web. Cellulosic fibers that may be incorporated into the web include but not limited to nonwoody fibers, such as cotton, abaca, kenaf, sabai grass, flax, esparto grass, straw, jute hemp, bagasse, milkweed floss fibers, and pineapple leaf fibers; and woody or pulp fibers such as those obtained from deciduous and coniferous trees, including softwood fibers, such as northern and southern softwood kraft fibers; hardwood fibers, such as eucalyptus, maple, birch, and aspen. Pulp fibers can be prepared in high-yield or low-yield forms and can be pulped in any known method, including kraft, sulfite, high-yield pulping methods and other known pulping methods. Fibers prepared from organosolv pulping methods can also be used.

In one aspect, the tissue web may be made exclusively from cellulosic fibers. Alternatively, cellulosic fibers can be combined with other fibers for increasing strength or for improving one or more properties. For example, the web can also contain any suitable synthetic polymer fiber or any suitable regenerated cellulose fiber. Exemplary polymer fibers that may be incorporated in the web include, for instance, polyester fibers, polyolefin fibers such as polyethylene fibers and/or polypropylene fibers, and mixtures thereof. The polymer fibers can also be bicomponent fibers that include a sheath-core configuration or a side-by-side configuration.

Synthetic cellulose fiber types include rayon in all its varieties and other fibers derived from viscose or chemically-modified cellulose. Chemically treated natural cellulosic fibers can be used such as mercerized pulps, chemically stiffened or crosslinked fibers, or sulfonated fibers. For good mechanical properties in using papermaking fibers, it can be desirable that the fibers be relatively undamaged and largely unrefined or only lightly refined. While recycled fibers can be used, virgin fibers are generally useful for their mechanical properties and lack of contaminants. Mercerized fibers, regenerated cellulosic fibers, cellulose produced by microbes, rayon, and other cellulosic material or cellulosic derivatives can be used. In certain embodiments capable of high bulk and good compressive properties, the fibers can have a Canadian Standard Freeness of at least 200, more specifically at least 300, more specifically still at least 400, and most specifically at least 500.

In addition to fibers, the web can also contain various other different components and chemicals. For instance, the web can also contain a debonding agent, humectants and plasticizers such as low molecular weight polyethylene glycols and polyhydroxy compounds such as glycerin and propylene glycol. Materials that supply skin health benefits such as mineral oil, aloe extract, vitamin E, silicone, lotions in general and the like may also be incorporated into the finished products.

In general, the products of the present disclosure can be used in conjunction with any known materials and chemicals that are not antagonistic to its intended use. Examples of such materials include but are not limited to odor control agents, such as odor absorbents, activated carbon fibers and particles, baby powder, baking soda, chelating agents, zeolites, perfumes or other odor-masking agents, cyclodextrin compounds, oxidizers, and the like. Superabsorbent particles may also be employed. Additional options include cationic dyes, optical brighteners, humectants, emollients, and the like.

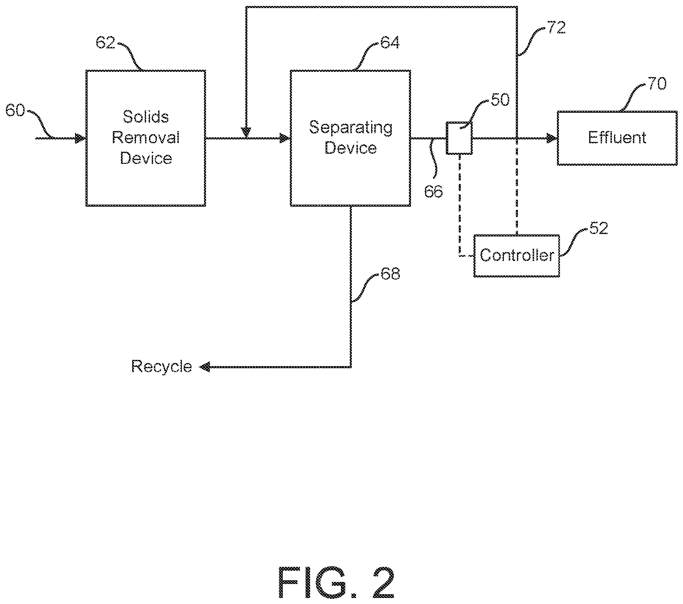

In order to form the base web, the aqueous solution is combined with a selected fiber furnish in conjunction with any auxiliary agents. The suspension of fibers is then pumped to a tank and from the tank is fed to a headbox. **FIGS. 1** and **2****,** for instance, show one embodiment of a process in accordance with the present disclosure for forming the web.

Referring to **FIG. 1****,** the aqueous suspension of fibers as described above is formed in a tank **12.** As shown in **FIG. 1****,** for instance, the tank **12** can be in communication with a water supply **22** for feeding water to the tank and a surfactant supply **24** for feeding a surfactant to the tank **12.** The fiber furnish is fed to the tank **12** and combined with the water and surfactant. In one aspect, an aqueous suspension of fibers is formed that is primarily in liquid form. Alternatively, the aqueous solution formed by combining the surfactant and water can be agitated and formed into a foam for forming a foamed suspension of fibers. One or more surfactants are used in either process.

From the headbox **10,** the fiber suspension is issued onto an endless traveling forming fabric **26** supported and driven by rolls **28** in order to form a wet embryonic web **12.** As shown in **FIG. 1****,** a forming board **14** may be positioned below the web **12** adjacent to the headbox **10.** Once formed on the forming fabric **26,** the formed web can have a consistency of less than about 50%, such as less than about 20%, such as less than about 10%, such as less than about 5%. In fact, the forming consistency can be less than about 2%, such as less than about 1.8%, such as less than about 1.5%. The forming consistency is generally greater than about 0.5%, such as greater than about 0.8%.

Once the wet web is formed on the forming fabric **26,** the web is conveyed downstream and dewatered. For instance, the process can optionally include a plurality of vacuum devices **16,** such as vacuum boxes and vacuum rolls. The vacuum boxes assist in removing moisture from the newly formed web **12.**

As shown in **FIG. 1****,** the forming fabric **26** may also be placed in communication with a steambox **18** positioned above a pair of vacuum rolls **20.** The steambox **18,** for instance, can increase dryness and reduce cross-directional moisture variance. The applied steam from the steambox **18** heats the moisture in the wet web **12** causing the water in the web to drain more readily, especially in conjunction with the vacuum rolls **20.** From the forming fabric **26,** the newly formed web **12** is conveyed downstream and dried. The web can be dried using any suitable drying device. For instance, the web can be through-air dried or placed on a heated drying drum and creped or left uncreped. In **FIG. 1****,** for instance, the formed web **12** is placed in contact with two heated drying drums **38** and **40.** In one embodiment, from the drying drums **38** and **40,** the web can be fed to a through-air dryer prior to being wound into a roll.

The process of the present disclosure can produce webs with good bulk characteristics. The sheet "bulk" is calculated as the quotient of the caliper of a dry tissue sheet, expressed in microns, divided by the dry basis weight, expressed in grams per square meter. The resulting sheet bulk is expressed in cubic centimeters per gram. More specifically, the caliper is measured as the total thickness of a stack of ten representative sheets and dividing the total thickness of the stack by ten, where each sheet within the stack is placed with the same side up. Caliper is measured in accordance with TAPPI test method T411 om-89 "Thickness (caliper) of Paper, Paperboard, and Combined Board" with Note 3 for stacked sheets. The micrometer used for carrying out T411 om-89 is an Emveco 200-A Tissue Caliper Tester available from Emveco, Inc., Newberg, Oreg. The micrometer has a load of 2.00 kilo-Pascals (132 grams per square inch), a pressure foot area of 2500 square millimeters, a pressure foot diameter of 56.42 millimeters, a dwell time of 3 seconds and a lowering rate of 0.8 millimeters per second.

Tissue webs made according to the present disclosure can be used in all different types of products. For instance, the tissue web can be used to produce bath tissue, facial tissue, paper towels, industrial wipers, personal care products and the like.

In accordance with the present disclosure, the process and system illustrated in **FIG. 1** further includes analyzers and controls for determining surfactant concentration of the aqueous solution being fed to the tank **12** or within the tank **12.** For example, as shown in **FIG. 1****,** the tissue making system further includes a carbon parameter analyzer **50** that is configured to receive fluid samples from the tank **12** or from a fluid supply line to the tank **12.** In accordance with the present disclosure, the carbon parameter analyzer **50** is configured to measure a carbon related parameter within the aqueous solution that is either contained in the tank **12** or fed to the tank **12.** The carbon related parameter, for instance, can be Chemical Oxygen Demand (COD), Total Organic Carbon (TOC), or the like. The measured carbon parameter can then be correlated to a surfactant concentration contained within the aqueous solution. The carbon parameter analyzer **50** is also selected so that the analyzer can receive samples from the tank **12** and measure a carbon parameter at a relatively fast rate. The carbon parameter analyzer **50,** for instance, in one aspect, can measure a carbon parameter within the aqueous solution in an amount of time of less than about 2 hours, such as less than about 1 hour, such as less than about 30 minutes, such as less than about 20 minutes, such as less than about 10 minutes, such as less than about 5 minutes, such as less than about 3 minutes, such as less than about 2 minutes.

For example, in one embodiment, the carbon parameter analyzer **50** can measure COD or TOC using oxidation. Such instruments are currently commercially available and can measure a carbon parameter in less than about two minutes. For example, in one aspect, the carbon parameter analyzer **50** can use ferrate oxidation in order to measure COD and/or TOC levels in a fluid sample. When using an oxidative method, TOC and/or COD can be measured by first adding an acid reagent to the fluid sample to convert inorganic carbon in the sample to gaseous carbon dioxide. The carbon dioxide is removed by sparging the solution with a carbon dioxide-free carrier gas, such as nitrogen. The liberated carbon dioxide can be measured to determine Total Inorganic Carbon (TIC). A chemical oxidant is then added to the resulting solution to oxidize the organic carbon present in the sample. In one aspect, the oxidant comprises a ferrate. The organic carbon is converted into a carbonate species optionally with the aid of ultraviolet radiation. Carbon dioxide is then produced which is once again measured as TOC.

Carbon parameter analyzers that measure using oxidation, for instance, are commercially available from Hach. Suitable analyzers are also described in U.S. Patent No. 9,476,866 and U.S. Patent Publication No. 2015/0108009 which are both incorporated herein by reference.

In an alternative embodiment, the carbon parameter analyzer **50** can include a fluorescence sensor that measures a fluorescence intensity in order to determine a carbon parameter, such as COD, TOC, or even biological oxygen demand (BOD). In one aspect, the carbon parameter analyzer **50** can also include a temperature sensor and a turbidity sensor that is used in combination with the fluorescence sensor to determine a carbon parameter. Carbon parameter analyzers that are commercially available which use fluorescence and/or ultraviolet light to measure carbon parameters are available from Proteus, which is a division of R.S. Hydro Limited. An example of a carbon parameter analyzer is also disclosed in U.S. Patent Publication No. 2019/0242864, which is incorporated herein by reference.

As shown in **FIG. 1****,** the carbon parameter analyzer **50** is in communication with a controller **52.** The controller **52,** for instance, can be any suitable programmable device. For instance, the controller **52** can be a microprocessor or a plurality of microprocessors. In one aspect, the controller **52** can be a computer that receives information from the carbon parameter analyzer **50** either wirelessly or through wire means.

More particularly, the controller **52** is configured to receive measured carbon parameter data from the carbon parameter analyzer **50.** The controller **52** is then configured to calculate a surfactant concentration within the aqueous solution based upon the measured carbon parameter value, whether that value be COD, TOC, a combination of both, or some other suitable carbon parameter. In one aspect, the measured surfactant concentration can then be communicated to a user for possibly making adjustments to the process. For instance, based on the measured surfactant concentration, the surfactant supply **24,** the water supply **22,** or both can be adjusted in order to selectively increase or decrease surfactant concentration within the aqueous solution contained within the tank **12.**

In one aspect, the controller **52** is configured to automatically make adjustments to the system. For instance, the controller **52** can be in communication with the surfactant supply **24** and/or the water supply **22.** Based on the determined surfactant concentration, for instance, the controller **52** can selectively increase or decrease the flow rate of a surfactant into the process and/or selectively increase or decrease the flow rate of water into the process.

In one particular embodiment, the controller **52** can be programmed with preset surfactant concentration limits. For example, the controller **52** can store a first preset limit and a second preset limit. The determined surfactant concentration derived from one or more carbon parameter measurements received from the carbon parameter analyzer **50** can then be compared to the first and second preset limits to ensure that surfactant concentrations stay within a desired range. For example, the controller **52** can be configured to automatically compare the determined surfactant concentration to the preset limits to ensure that the surfactant concentration is above a first preset limit and below a second preset limit. Should the determined surfactant concentration be outside of the range set by the preset limits, the controller **52** can either notify a user in order to make adjustments or can automatically make adjustments within the process by controlling surfactant supply and/or water supply. The controller **52** can make adjustments to the process in an open loop manner or in a closed loop manner.

Through the process as shown in **FIG. 1****,** surfactant concentrations within the process are continuously monitored so that on the fly adjustments can be made within the process in order to ensure that surfactant concentrations stay within tightly constrained tolerances. The carbon parameter analyzer **50,** for instance, can take measurements for communication to the controller **52** at least every 12 hours, such as at least every 6 hours, such as at least every 4 hours, such as at least every hour. In one aspect, the carbon parameter analyzer **50** can be designed to take carbon parameter measurements every 45 minutes, such as every 30 minutes, such as every 20 minutes, such as every 10 minutes, such as even every 5 minutes.

The determination of surfactant concentration from the measured carbon parameter can be accomplished using various methods and techniques. In one aspect, for instance, the surfactant concentration can be mathematically estimated from the carbon parameter measurement based upon input of the various system variables.

In another aspect, reference data is inputted into the controller **52** for determining surfactant concentration. The reference data, for instance, can be experimentally obtained based upon process conditions. For example, stock surfactant solutions can be prepared at a known surfactant concentration. The stock solutions can be prepared so as to mirror the aqueous solution that is fed through the process illustrated in **FIG. 1****.** The surfactant concentration can be varied within the stock solutions based upon an estimated range of surfactant concentrations that may be encountered during running of the process illustrated in **FIG. 1****.** For each stock solution having a known surfactant concentration, a carbon parameter can be measured from the solution. The carbon parameter, for instance, can be COD, TOC or both. A calibration curve can then be created that compares surfactant concentration to the measured carbon parameter. In many processes, such as the one illustrated in **FIG. 1****,** it was discovered that the calibration curve is substantially linear. Discovering a linear relationship between the carbon parameter and surfactant concentrations in some systems facilitates adjustments for selectively increasing or decreasing surfactant concentrations. In other words, the linear relationship makes it easy to extrapolate future surfactant concentrations based on adjustments to the flow of surfactant and/or the flow of water into the system.

In **FIG. 1****,** the surfactant control system of the present disclosure is designed and used to maintain surfactant levels within preset limits as the surfactant is being fed into the process to produce the product. In other applications, however, the surfactant control system of the present disclosure can be used to maintain surfactant levels below a preset limit. For example, all of the fluids that are drained from the process illustrated in **FIG. 1** can be collected and fed to a process where the surfactant can be removed from the aqueous solution and/or to a process where the drain solution is monitored for surfactant concentration and, if below a certain level, can be either released to the environment as a wastewater or recycled to some other process within the plant. For example, although one or more surfactants can provide numerous advantages and benefits in the process illustrated in **FIG. 1****,** it may be undesirable for the surfactant to be present above certain concentrations in other parts of the process. In this regard, the present disclosure is also directed to a system and process for maintaining surfactant concentrations below a certain level in an effluent or process stream.

Referring to **FIG. 2****,** for instance, one embodiment of a system and method for controlling surfactant concentrations in an effluent stream is illustrated. As shown in **FIG. 2****,** a process stream **60** represents the initial stream being treated. The process stream **60,** for instance, can be an aqueous solution containing a surfactant. The process stream **60,** for instance, can be a waste stream or a byproduct stream produced in the process facility. The process stream **60,** for instance, can be a stream created by collecting all of the fluid runoff that is collected from the process illustrated in **FIG. 1****.**

As shown in **FIG. 2****,** the process stream **60** is optionally fed to a solids removal device **62.** The solids removal device **62** may be needed if the process stream **60** contains suspended solids. For instance, a fluid collected from the process illustrated in **FIG. 1** may contain residual amounts of papermaking fibers or other particulate material. In general, the solids removal device **62** can be any suitable device capable of removing suspended solids and may operate through filtration, flotation, settling, and combinations thereof.

In one aspect, for instance, the solids removal device can use dissolved air flotation. Dissolved air flotation clarifiers, for instance, typically receive the process fluid into a large tank that can be rectangular or circular in nature. The process fluid is introduced in a manner that reduces velocity and distributes the aqueous solution evenly. A side stream of water is pressurized and high pressure air is introduced into the side stream. A high concentration of air dissolves into the high pressure water stream. The high pressure water stream is then introduced into the process stream as it enters the dissolved air flotation clarifier. Release of the high pressure to atmospheric pressure allows the super saturated air to come out of solution creating microbubbles. The microbubbles attach to floc particles, which decreases the effective density of the floc particles, causing them to rise to the top of the process stream. The microbubbles/floc mat that forms at the top of the clarifier can then be mechanically scraped from the surface for further solids processing. Dissolved air flotation is particularly effective when removing paper making fibers from an aqueous solution.

In an alternative embodiment, the separating device 64 can operate using suspended air flotation. A suspended air flotation clarifier works similar to a dissolved air flotation clarifier, but instead forms electrostatically charged microbubbles produced at atmospheric pressure. The suspended air flotation clarifier produces a froth that contains greater than 40% air. Bubble formation within the suspended air flotation clarifier may provide advantages over dissolved air flotation. In particular, suspended air flotation clarifiers may produce smaller bubbles which increase the surface area available for attachment to the suspended solids. The smaller bubbles may produce a surface tension advantage which improves the ability of the suspended solids to stick to the bubbles and have a general resistance to coalescing of bubbles which increases bubble size thereby decreasing bubble surface area. Consequently, in some applications, suspended air flotation clarifiers may have increased rise rates resulting in smaller footprints.

In still another embodiment, the solids removal device **62** may comprise a disc filter system. Disc filters include a filtration skid containing multiple filter pods. Each pod contains disc filters. The disc filters are stacked together in each pod and may be compressed with springs when in filtration mode for removal of suspended solids. As solids accumulate in the filters, differential pressure increases across the filter as the solids become trapped in the open areas of the filter. After a particular differential pressure has been achieved or after a certain amount of time, the disc filters can be backwashed to clean out the captured solids. During backwash, the compression from the springs is released while reverse flow of water is introduced tangentially to the discs. The discs spin freely, releasing solids. If desired, certain pods can be operating in backwash mode while other pods can be operating in filter mode.

Prior to feeding the process stream **60** to the solids removal device **62,** the process stream may need to be pretreated in certain applications. For example, depending upon the amount of surfactant contained in the aqueous solution, the process stream **60** may be pretreated with a defoamer and may undergo a pH treatment.

As shown in **FIG. 2****,** after the solids removal device **62,** the process stream **60** is fed to a separating device **64.** The separating device **64** is for removing and separating surfactants from the process stream **60.** In one aspect, the separating device can be used to degrade or breakdown the surfactants so that the components are released into the environment. Alternatively, the surfactants can be removed from the process stream **60.**

In one aspect, for instance, the separating device **64** can use dissolved ozone flotation. Dissolved ozone flotation, for instance, combines the solids removal device **62** with the separating device **64** for removing suspended solids and also degrading surfactants contained in the process stream **60.**

During dissolved ozone flotation, solids are floated to the top of the dissolved ozone flotation clarifier for removal. During the process, ozone is also combined with the pressurized air to form the bubbles. The ozone provides a mechanism for oxidization of surfactants in the process stream **60.** For instance, during dissolved ozone flotation, surfactants and other carbon-based materials are exposed to high concentrations of ozone, causing oxidation of the materials.

In another embodiment, the separating device **64** can be any suitable filter device, such as a carbon filter that uses granular activated carbon. Granular activated carbon is a media that has adsorptive properties for organic compounds. The media is generated from various materials, including coal, coconut shells, wood, and the like. The process stream **60,** for instance, can be introduced to the top of the granular activated carbon filter media vessel and flowed downward through the device. The granular activated carbon is held in place with an underdrain that prevents the media from migrating downstream of the reaction vessel and allows reverse, upward flow for backwashing accumulated solids. Once the granular activated carbon media is exhausted, the material is removed and replaced with fresh media. The exhausted media can be regenerated. Granular activated carbon filters are well suited to removing surfactants from a process stream, such as process stream **60.**

In one aspect, the separating device **64** operates using reverse osmosis. Reverse osmosis devices utilize a partially permeable membrane to remove ions and molecules from water. Removal is accomplished through the application of high pressure to the water as it is introduced into the membrane modules. For instance, pressures used during reverse osmosis can be greater than about 200 psi, such as greater than about 500 psi, such as greater than about 800 psi, such as greater than about 1000 psi, such as greater than about 1200 psi, and generally less than about 2000 psi, such as less than about 1500 psi. The pressure forces the process stream **60** through the membrane. Reverse osmosis devices produce a surfactant poor stream **66** and a surfactant rich stream **68** as shown in **FIG. 2****.**

As shown in **FIG. 2****,** the surfactant poor stream **66** exiting the separating device **64** is in communication with the carbon parameter analyzer **50.** The carbon parameter analyzer **50,** for instance, can periodically remove fluid samples from the surfactant poor stream **66** and analyze the samples for a carbon parameter. The carbon parameter analyzer **50** can be in communication with the controller **52.** The controller **52** can be configured to receive measurement data from the carbon parameter analyzer **50.** The controller **52** can then determine a surfactant concentration within the surfactant poor stream **66** based upon the measured carbon parameter. In the embodiment illustrated in **FIG. 2****,** the controller **52** can be designed to determine when surfactant levels are above a preset limit. For example, one of the purposes of the process illustrated in **FIG. 2** is to maintain surfactant concentrations below a particular level prior to releasing the surfactant poor stream **66** to the effluent **70.**

In one aspect, the controller can determine the surfactant concentration within the surfactant poor stream **66** and, if the surfactant concentration is below the preset limit, permit the surfactant poor stream **66** to enter the effluent **70.** The preset limit, for example, can be less than 100 ppm, such as less than 80 ppm, such as less than 40 ppm, such as less than 20 ppm. If the determined surfactant concentration is above the preset limit, on the other hand, the controller **52** can be configured to take remedial action. For example, as shown in **FIG. 2****,** in one embodiment, the surfactant poor stream **66** can enter a recycle loop **72** and fed back to the separating device **64.** The controller **52,** for instance, can be designed to control a valve that causes the surfactant poor stream **66** to either enter the effluent stream **70** or the recycle stream **72.**

In another embodiment, the controller **52** can be in communication with a holding tank and can send the surfactant poor stream **66** to the holding tank if surfactant concentration levels are above the preset limit. The surfactant poor stream **66** fed to the holding tank can then be further treated or diluted in order to reduce surfactant levels.

The effluent **70** containing surfactant concentrations below a desired level can then be released to the environment as a wastewater and/or recycled and used as a makeup water stream in the process facility.

The present disclosure may be better understood with reference to the following example.

### EXAMPLE

Various different surfactant and water stock solutions were prepared and tested for COD levels. The data was then used to create calibration curves.

In this example, three different sets of samples were produced. Each set of samples was produced for a different concentration range. In the first set of samples, the concentration range was from 200 to 15,000 mg/L COD and from 0 to 2,000 ppm surfactant. During all the tests, a Hach DR3900 carbon parameter analyzer was used according to manufacturer method 435 for the first and third set of samples. Manufacturer method 430 was used for the second set of samples due to the lower concentrations. In each of the sets of samples, a C8 and C10 alkyl polyglycoside surfactant was used.

During each set of samples, stock solutions were prepared and tested to determine surfactant concentration. COD was also measured three times for each sample and averaged. Filtered tap water was combined with the surfactant in each set of samples.

For the first set of samples, the following results were obtained. In the first set of samples, the read out value was 1/10 the actual value and use to construct the calibration curve.

| Sample No. | Surfactant Target ppm | Surfactant Actual ppm | COD (mg/L) (actual) | COD (mg/L) (read out value) |
|---|---|---|---|---|
| 1 | 0 | 0.0 | 0 | 0 |
| 2 | 500 | 524.4 | 1920 | 192 |
| 3 | 1000 | 1063.1 | 3910 | 391 |
| 4 | 1500 | 1573.1 | 5780 | 578 |
| 5 | 2000 | 2022.2 | 7300 | 730 |

For the second set of samples, the surfactant concentration range was from 0 to 70 ppm and the COD measurement range was from 3 mg/L to 150 mg/L. The following results were obtained:

| Sample No. | Surfactant Target ppm | Surfactant Actual ppm | COD (mg/L) |
|---|---|---|---|
| 1 | 0 | 0.0 | 0 |
| 2 | 5 | 9.8 | 12 |
| 3 | 25 | 37.3 | 66 |
| 4 | 50 | 62.3 | 124 |
| 5 | 70 | 74.7 | 129 |

For the third set of samples, the surfactant concentration range was from 0 to 750 ppm and the COD measurement range was from 20 mg/L to 1500 mg/L. The following results were obtained:

| Sample No. | Surfactant Target ppm | Surfactant Actual ppm | COD (mg/L) |
|---|---|---|---|
| 1 | 0 | 0.0 | -2 |
| 2 | 50 | 49.6 | 95 |
| 3 | 250 | 249.3 | 443 |
| 4 | 500 | 537.2 | 978 |
| 5 | 750 | 775.0 | 1421 |

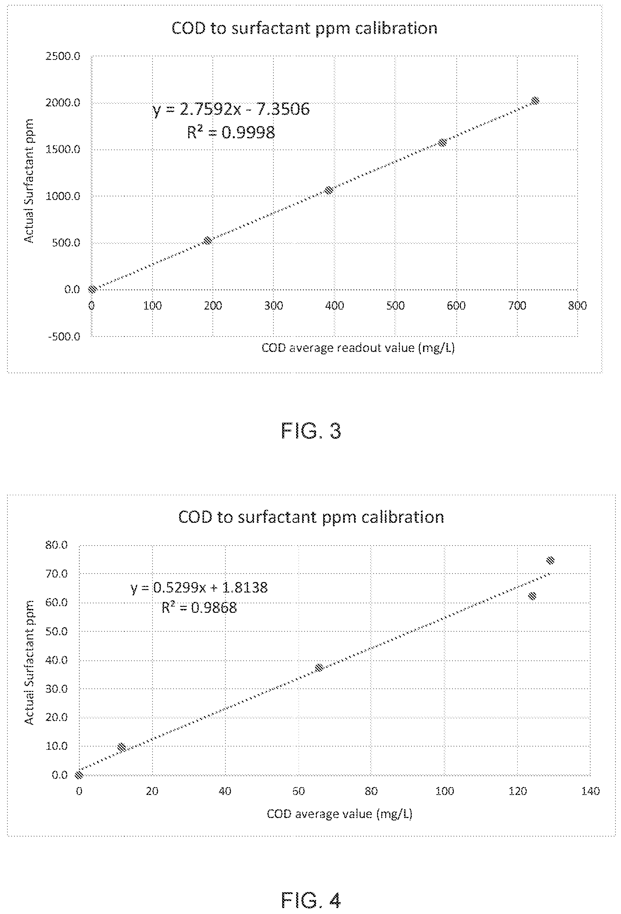

The results of the above three sets of samples are illustrated in **FIGS. 1****,** **2** and **3****.** As shown, each set of samples produced a linear correlation graph. The results illustrate a clear relationship between the measured COD value and surfactant concentration.

These and other modifications and variations to the present invention may be practiced by those of ordinary skill in the art, without departing from the scope of the appended claims. Furthermore, those of ordinary skill in the art will appreciate that the foregoing description is by way of example only, and is not intended to limit the invention so further described in such appended claims.

## Claims

1. A method for determining surfactant levels in a fluid comprising:
collecting a fluid sample from an aqueous solution, the aqueous solution containing a surfactant; measuring a carbon related parameter in the fluid sample, the carbon related parameter comprising Chemical Oxygen Demand or Total Organic Carbon; and
determining a surfactant concentration in the aqueous solution by comparing the measured carbon related parameter to a calibration data that indicates surfactant concentration or calculating the surfactant concentration based on the measured carbon related parameter;
**characterised in that** the method further comprises the step of feeding the aqueous solution through a separating device (64) that removes surfactant from the aqueous solution prior to collecting the fluid sample.

2. A method as defined in claim 1, wherein, based on the determined surfactant concentration, the method further includes the step of increasing or decreasing surfactant concentration in the aqueous solution in order to maintain surfactant concentration within a preset limit.

3. A method as defined in any of the preceding claims, wherein the aqueous solution comprises a fluid stream in an industrial process, preferably wherein the industrial process is a papermaking process, a tissue making process, or a process for producing an absorbent article.

4. A method as defined in claim 2, wherein the measured carbon related parameter is fed to a controller for determining the surfactant concentration preferably wherein, based on the determined surfactant concentration, the controller selectively increases or decreases surfactant concentration in the aqueous solution in order to maintain the surfactant concentration within a preset limit.

5. A method as defined in claim 2 or claim 4, wherein the surfactant concentration is selectively decreased in order to maintain the surfactant concentration below a present limit, or wherein the surfactant concentration is selectively increased or decreased in order to maintain the surfactant concentration above a first preset limit and below a second preset limit.

6. A method as defined in any of the preceding claims, wherein fluid samples are collected and the carbon related parameter is measured at least every 12 hours, such as at least every 6 hours, such as at least every 4 hours, such as at least every hour; and/or
wherein the surfactant contained in the aqueous solution is a nonionic surfactant, preferably wherein the surfactant comprises sodium dodecyl sulfate, ammonium lauryl sulfate, a fatty acid amine, an amine oxide, a fatty acid quaternary compound, an alkyl polyglycoside, or lauryl sulfate; and/or
wherein the aqueous solution comprises a foamed suspension containing greater than about 30% air by volume.

7. A method as defined in claim 2 or 4, wherein the aqueous solution is being fed to a headbox for forming a suspension of particulate material, the aqueous solution being formed by combining water from a water supply with a surfactant from a surfactant supply, and wherein the surfactant concentration is selectively increased or decreased by increasing or decreasing an amount of the surfactant from the surfactant supply being combined with the water from the water supply.

8. A method as defined in claim 1, wherein the separating device produces a surfactant poor stream (66) containing the aqueous solution and a surfactant rich stream (68).

9. A method as defined in claim 8, further comprising the step of recycling the surfactant rich stream back into an industrial process and feeding the surfactant poor stream to an effluent (70).

10. A method as defined in claim 8 or 9, wherein if the determined surfactant concentration is above the preset limit, the surfactant concentration is decreased in the aqueous solution by feeding the surfactant poor stream back into the separating device; and/or
wherein if the surfactant concentration is below the preset limit, the surfactant poor stream is permitted to enter an effluent;
optionally wherein the preset limit is less than 100 ppm.

11. A method as defined in any of claims 1 or 8-10, further comprising the step of filtering the aqueous solution to remove solids prior to feeding the aqueous solution through the separating device.

12. A method as defined in claim 11, wherein the aqueous solution is filtered using dissolved air flotation or suspended air flotation.

13. A method as defined in any of claims 1 or 8-12, wherein the separating device operates using dissolved ozone flotation; or wherein the separating device comprises a filter device; or wherein the separating device comprises an activated carbon filter; or wherein the separating device operates using reverse osmosis.

14. A system for determining surfactant concentration comprising:
a carbon parameter analyzer (50) that measures Chemical Oxygen Demand or Total Organic Carbon in a fluid sample, the carbon parameter analyzer being configured to receive a fluid sample from an industrial fluid stream, the industrial fluid stream comprising an aqueous solution containing a surfactant;
a controller (52) in communication with the carbon parameter analyzer for receiving measured Chemical Oxygen Demand data or measured Total Organic Carbon data from the carbon parameter analyzer, the controller being configured to determine a surfactant concentration in the fluid sample based on the measured Chemical Oxygen Demand or the measured Total Organic Carbon received from the carbon parameter analyzer; and
**characterised in that** the system comprises a separating device (64) configured to remove surfactant from the aqueous solution

15. A system as defined in claim 14, wherein the controller is configured to control flow of a surfactant or flow of a water supply into the industrial process in order to selectively increase or decrease surfactant concentration in the aqueous solution, preferably wherein the carbon parameter analyzer uses ferrate oxidation to measure Chemical Oxygen Demand or Total Organic Carbon, or wherein the carbon parameter analyzer comprises a fluorescence sensor.

## Patentansprüche

1. Verfahren zum Bestimmen des Tensidgehalts in einem Fluid, umfassend:
Auffangen einer Fluidprobe aus einer wässrigen Lösung, wobei die wässrige Lösung ein Tensid enthält; Messen eines kohlenstoffbezogenen Parameters in der Fluidprobe, der kohlenstoffbezogene Parameter umfassend einen chemischen Sauerstoffbedarf oder einen gesamten organischen Kohlenstoff; und
Bestimmen einer Tensidkonzentration in der wässrigen Lösung durch Vergleich des gemessenen kohlenstoffbezogenen Parameters mit Kalibrierdaten, die die Tensidkonzentration angeben, oder Berechnen der Tensidkonzentration basierend auf dem gemessenen kohlenstoffbezogenen Parameter;
**dadurch gekennzeichnet, dass** das Verfahren ferner den Schritt des Einspeisens der wässrigen Lösung durch eine Trennvorrichtung (64), die Tensid aus der wässrigen Lösung vor dem Auffangen der Fluidprobe entfernt, umfasst.

2. Verfahren nach Anspruch 1, wobei das Verfahren, basierend auf der bestimmten Tensidkonzentration, ferner den Schritt des Erhöhens oder Verringerns der Tensidkonzentration in der wässrigen Lösung einschließt, um die Tensidkonzentration innerhalb eines voreingestellten Grenzwerts zu halten.

3. Verfahren nach einem der vorstehenden Ansprüche, wobei die wässrige Lösung einen Fluidstrom in einem industriellen Prozess umfasst, vorzugsweise wobei der industrielle Prozess ein Papierherstellungsprozess, ein Papiertaschentuchherstellungsprozess oder ein Prozess zum Herstellen eines absorbierenden Artikels ist.

4. Verfahren nach Anspruch 2, wobei der gemessene kohlenstoffbezogene Parameter in eine Steuerung eingespeist wird, zum Bestimmen der Tensidkonzentration, vorzugsweise wobei, basierend auf der bestimmten Tensidkonzentration, die Steuerung die Tensidkonzentration in der wässrigen Lösung wahlweise erhöht oder verringert, um die Tensidkonzentration innerhalb eines voreingestellten Grenzwerts zu halten.

5. Verfahren nach Anspruch 2 oder 4, wobei die Tensidkonzentration wahlweise verringert wird, um die Tensidkonzentration unterhalb eines aktuellen Grenzwerts zu halten, oder wobei die Tensidkonzentration wahlweise erhöht oder verringert wird, um die Tensidkonzentration oberhalb eines ersten voreingestellten Grenzwerts und unterhalb eines zweiten voreingestellten Grenzwerts zu halten.

6. Verfahren nach einem der vorstehenden Ansprüche, wobei Fluidproben aufgefangen werden und der kohlenstoffbezogene Parameter mindestens alle 12 Stunden, wie mindestens alle 6 Stunden, wie mindestens alle 4 Stunden, wie mindestens stündlich gemessen wird; und/oder
wobei das Tensid, das in der wässrigen Lösung enthalten ist, ein nichtionisches Tensid ist, vorzugsweise wobei das Tensid Natriumdodecylsulfat, Ammoniumlaurylsulfat, ein Fettsäureamin, ein Aminoxid, eine quaternäre Fettsäureverbindung, ein Alkylpolyglycosid oder Laurylsulfat umfasst; und/oder
wobei die wässrige Lösung eine geschäumte Suspension, die mehr als etwa 30 Vol.-% Luft enthält, umfasst.

7. Verfahren nach Anspruch 2 oder 4, wobei die wässrige Lösung in einen Stoffauflaufkasten zum Ausbilden einer Suspension von partikulärem Material eingespeist wird, wobei die wässrige Lösung durch Mischen von Wasser aus einer Wasserversorgung mit einem Tensid aus einer Tensidversorgung ausgebildet wird, und wobei die Tensidkonzentration wahlweise erhöht oder verringert wird, durch Erhöhen oder Verringern einer Menge des Tensids aus der Tensidversorgung, die mit dem Wasser aus der Wasserversorgung gemischt wird.

8. Verfahren nach Anspruch 1, wobei die Trennvorrichtung einen tensidarmen Strom (66), der die wässrige Lösung enthält, und einen tensidreichen Strom (68) herstellt.

9. Verfahren nach Anspruch 8, ferner umfassend den Schritt des Rückführens des tensidreichen Stroms zurück in einen industriellen Prozess und des Einspeisens des tensidarmen Stroms in ein Abwasser (70).

10. Verfahren nach Anspruch 8 oder 9, wobei, falls die bestimmte Tensidkonzentration über dem voreingestellten Grenzwert liegt, die Tensidkonzentration in der wässrigen Lösung durch Rückeinspeisen des tensidarmen Stroms in die Trennvorrichtung verringert wird; und/oder
wobei, falls die Tensidkonzentration unterhalb des voreingestellten Grenzwerts liegt, der tensidarme Strom in ein Abwasser eingeleitet werden darf;
optional, wobei der voreingestellte Grenzwert weniger als 100 ppm beträgt.

11. Verfahren nach einem der Ansprüche 1 oder 8 bis 10, ferner umfassend den Schritt des Filtrierens der wässrigen Lösung, um Feststoffe vor dem Einspeisen der wässrigen Lösung durch die Trennvorrichtung zu entfernen.

12. Verfahren nach Anspruch 11, wobei die wässrige Lösung unter Verwendung von Flotation mit gelöster Luft oder Flotation mit suspendierter Luft filtriert wird.

13. Verfahren nach einem der Ansprüche 1 oder 8 bis 12, wobei die Trennvorrichtung unter Verwendung von Flotation mit gelöstem Ozon arbeitet; oder wobei die Trennvorrichtung eine Filtervorrichtung umfasst; oder wobei die Trennvorrichtung einen Aktivkohlefilter umfasst; oder bei der die Trennvorrichtung unter Verwendung von Umkehrosmose arbeitet.

14. System zum Bestimmen der Tensidkonzentration, umfassend:
ein Kohlenstoffparameteranalysator (50), der den chemischen Sauerstoffbedarf oder den gesamten organischen Kohlenstoff in einer Fluidprobe misst, wobei der Kohlenstoffparameteranalysator konfiguriert ist, um eine Fluidprobe aus einem industriellen Fluidstrom zu empfangen, der industrielle Fluidstrom umfassend eine wässrige Lösung, die ein Tensid enthält;
eine Steuerung (52), die mit dem Kohlenstoffparameteranalysator in Verbindung steht, zum Empfangen von Daten des gemessenen chemischen Sauerstoffbedarfs oder von Daten des gemessenen gesamten organischen Kohlenstoffs von dem Kohlenstoffparameteranalysator, wobei die Steuerung konfiguriert ist, um eine Tensidkonzentration in der Fluidprobe basierend auf dem gemessenen chemischen Sauerstoffbedarf oder dem gemessenen gesamten organischen Kohlenstoff, die von dem Kohlenstoffparameteranalysator empfangen werden, zu bestimmen; und
**dadurch gekennzeichnet, dass** das System eine Trennvorrichtung (64), die konfiguriert ist, um Tenside aus der wässrigen Lösung zu entfernen, umfasst.

15. System nach Anspruch 14, wobei die Steuerung konfiguriert ist, um den Fluss eines Tensids oder den Fluss einer Wasserzufuhr in den industriellen Prozess zu steuern, um die Tensidkonzentration in der wässrigen Lösung wahlweise zu erhöhen oder zu verringern, vorzugsweise wobei der Kohlenstoffparameteranalysator die Ferratoxidation verwendet, um den chemischen Sauerstoffbedarf oder den gesamten organischen Kohlenstoff zu messen, oder wobei der Kohlenstoffparameteranalysator einen Fluoreszenzsensor umfasst.

## Revendications

1. Procédé permettant de déterminer des niveaux de tensioactif dans un fluide, comprenant :
la collecte d'un échantillon de fluide à partir d'une solution aqueuse, la solution aqueuse contenant un tensioactif ; la mesure d'un paramètre lié au carbone dans l'échantillon de fluide, le paramètre lié au carbone comprenant la demande chimique en oxygène ou le carbone organique total ; et
la détermination d'une concentration de tensioactif dans la solution aqueuse par comparaison du paramètre lié au carbone mesuré à une donnée d'étalonnage qui indique la concentration de tensioactif ou par calcul de la concentration de tensioactif sur la base du paramètre lié au carbone mesuré ;
**caractérisé en ce que** le procédé comprend en outre l'étape consistant à faire passer la solution aqueuse dans un dispositif de séparation (64) qui élimine le tensioactif de la solution aqueuse avant de collecter l'échantillon de fluide.

2. Procédé selon la revendication 1, dans lequel, sur la base de la concentration de tensioactif déterminée, le procédé comporte en outre l'étape consistant à augmenter ou à diminuer la concentration de tensioactif dans la solution aqueuse afin de maintenir la concentration de tensioactif dans une limite prédéfinie.

3. Procédé selon l'une quelconque des revendications précédentes, dans lequel la solution aqueuse comprend un flux de fluide dans un processus industriel, de préférence dans lequel le processus industriel est un processus de fabrication de papier, un processus de fabrication de papiers sanitaires et domestiques ou un processus de production d'un article absorbant.

4. Procédé selon la revendication 2, dans lequel le paramètre lié au carbone mesuré est transmis à un contrôleur pour déterminer la concentration de tensioactif, de préférence dans lequel, sur la base de la concentration de tensioactif déterminée, le contrôleur augmente ou diminue sélectivement la concentration de tensioactif dans la solution aqueuse afin de maintenir la concentration de tensioactif à l'intérieur dans une limite prédéfinie.

5. Procédé selon la revendication 2 ou la revendication 4, dans lequel la concentration de tensioactif est sélectivement diminuée afin de maintenir la concentration de tensioactif en dessous d'une limite prédéfinie, ou dans lequel la concentration de tensioactif est sélectivement augmentée ou diminuée afin de maintenir la concentration de tensioactif au-dessus d'une première limite prédéfinie et en dessous d'une seconde limite prédéfinie.

6. Procédé selon l'une quelconque des revendications précédentes, dans lequel des échantillons de fluide sont collectés et le paramètre lié au carbone est mesuré au moins toutes les 12 heures, par exemple au moins toutes les 6 heures, par exemple au moins toutes les 4 heures, par exemple au moins toutes les heures ; et/ou
dans lequel le tensioactif contenu dans la solution aqueuse est un tensioactif non ionique, de préférence dans lequel le tensioactif comprend le dodécylsulfate de sodium, l'ammonium lauryl sulfate, une amine d'acide gras, un oxyde d'amine, un composé quaternaire d'acide gras, un polyglycoside d'alkyle, ou le lauryl sulfate ; et/ou
dans lequel la solution aqueuse comprend une suspension moussée contenant plus de 30 % environ d'air en volume.

7. Procédé selon la revendication 2 ou 4, dans lequel la solution aqueuse est acheminée vers une caisse de tête pour former une suspension de matière particulaire, la solution aqueuse étant formée par combinaison de l'eau provenant d'une alimentation en eau avec un tensioactif provenant d'une alimentation en tensioactif, et dans lequel la concentration de tensioactif est sélectivement augmentée ou diminuée par augmentation ou diminution d'une quantité du tensioactif provenant de l'alimentation en tensioactif qui est combinée à l'eau provenant de l'alimentation en eau.

8. Procédé selon la revendication 1, dans lequel le dispositif de séparation produit un flux pauvre en tensioactif (66) contenant la solution aqueuse et un flux riche en tensioactif (68).

9. Procédé selon la revendication 8, comprenant en outre l'étape consistant à recycler le flux riche en tensioactif dans un processus industriel et à acheminer le flux pauvre en tensioactif vers un effluent (70).

10. Procédé selon la revendication 8 ou 9, dans lequel, si la concentration de tensioactif déterminée est supérieure à la limite prédéfinie, la concentration de tensioactif est diminuée dans la solution aqueuse en renvoyant le flux pauvre en tensioactif dans le dispositif de séparation ; et/ou
dans lequel, si la concentration de tensioactif est inférieure à la limite prédéfinie, le flux pauvre en tensioactif est autorisé à entrer dans un effluent ;
éventuellement, dans lequel la limite prédéfinie est inférieure à 100 ppm.

11. Procédé selon l'une quelconque des revendications 1 ou 8 à 10, comprenant en outre l'étape consistant à filtrer la solution aqueuse pour éliminer des solides avant de faire passer la solution aqueuse dans le dispositif de séparation.

12. Procédé selon la revendication 11, dans lequel la solution aqueuse est filtrée par flottation à air dissous ou par flottation à air suspendu.

13. Procédé selon l'une quelconque des revendications 1 ou 8 à 12, dans lequel le dispositif de séparation fonctionne par flottation à l'ozone dissous ; ou dans lequel le dispositif de séparation comprend un dispositif de filtration ; ou dans lequel le dispositif de séparation comprend un filtre à charbon actif ; ou dans lequel le dispositif de séparation fonctionne par osmose inverse.

14. Système permettant de déterminer la concentration de tensioactif, comprenant :
un analyseur de paramètres de carbone (50) qui mesure la demande chimique en oxygène ou le carbone organique total dans un échantillon de fluide, l'analyseur de paramètres de carbone étant configuré pour recevoir un échantillon de fluide provenant d'un flux de fluide industriel, le flux de fluide industriel comprenant une solution aqueuse contenant un tensioactif ;
un contrôleur (52) en communication avec l'analyseur de paramètres de carbone pour recevoir des données de demande chimique en oxygène mesurées ou des données de carbone organique total mesurées en provenance de l'analyseur de paramètres de carbone, le contrôleur étant configuré pour déterminer une concentration de tensioactif dans l'échantillon de fluide sur la base de la demande chimique en oxygène mesurée ou du carbone organique total mesuré reçu(e) de l'analyseur de paramètres de carbone ; et
**caractérisé en ce que** le système comprend un dispositif de séparation (64) configuré pour éliminer le tensioactif de la solution aqueuse.

15. Système selon la revendication 14, dans lequel le contrôleur est configuré pour réguler le débit d'un tensioactif ou le débit d'une alimentation en eau dans le processus industriel afin d'augmenter ou de diminuer sélectivement la concentration de tensioactif dans la solution aqueuse, de préférence dans lequel l'analyseur de paramètres de carbone utilise l'oxydation de ferrate pour mesurer la demande chimique en oxygène ou le carbone organique total, ou dans lequel l'analyseur de paramètres de carbone comprend un capteur de fluorescence.
